Europäisches Patentamt

(19) European Patent Office

Office européen des brevets

(11) Publication number: **0 151 996 B1**

# EUROPEAN PATENT SPECIFICATION

(12)

(45) Date of publication of patent specification: **03.04.91**   (51) Int. Cl.⁵: **C12N 9/64, A61K 37/54**

(21) Application number: **85100804.5**

(22) Date of filing: **26.01.85**

(54) Process for the Preparation of a double-chain plasminogen activator.

(30) Priority: **30.01.84 JP 13455/84**
**30.01.84 JP 13456/84**

(43) Date of publication of application:
**21.08.85 Bulletin 85/34**

(45) Publication of the grant of the patent:
**03.04.91 Bulletin 91/14**

(84) Designated Contracting States:
**BE CH DE FR GB IT LI NL SE**

(56) References cited:
**EP-A- 41 766      EP-A- 0 099 126**
**EP-A- 0 100 982      EP-A- 0 124 613**
**WO-A-83/03101      US-A- 4 381 346**

**CHEMICAL ABSTRACTS, vol. 92, no. 25, June 23, 1980, p. 214, ref.no. 210724q, Columbus, Ohio, US; D. Vetterlein et al.:"Immunological quantitation and immunoadsorption of urokinase-like plasminogen activators secreted by human cells"**

**J.Biol.Chem., vol. 256 (1981), pp. 7035-7041**

**J.Biol.Chem., vol. 255 (1980), pp. 3665-3672**

(73) Proprietor: **Asahi Kasei Kogyo Kabushiki Kaisha**
**2-6, Dojimahama 1-chome Kita-ku**
**Osaka-shi Osaka 530(JP)**

Proprietor: **KOWA COMPANY, LTD.**
**6-29, 3-chome Nishiki**
**Naka-ku Nagoya-shi Aichi-ken(JP)**

(72) Inventor: **Mori, Toshihito**
**4-16-3, Fujimi-cho**
**Higashimurayama-shi Tokyo(JP)**
Inventor: **Yoshizaki, Hideo**
**Sayamadai Hai-copo 506 1-4-9, Sayamadai**
**Sayama-shi Saitama(JP)**
Inventor: **Hasegawa, Akio**
**5-24, Sugizaki-cho**
**Numazu-shi Shizuoka(JP)**

(74) Representative: **Werner, Hans-Karsten, Dr. et al**
**Deichmannhaus am Hauptbahnhof**
**W-5000 Köln 1(DE)**

## Description

This invention relates to a process for the production of purified plasminogen activators. More specifically, this invention relates to the production in pure form of a double-chain plasminogen activator collected from a tissue cultured liquor of human normal tissue derived cells.

At present, urokinase separated and purified from urine or cultured liquor of kidney cells and streptokinase collected from cultured liquor of Streptococci are plasminogen activators employed in practical use as thrombolytic agents.

However, since urokinase and streptokinase possess poor affinity to fibrin, it is frequently necessary to administer them in large amounts in order to obtain the required effect on treatment. When large doses are administered, side effects such as gastro internal hemorrhage are manifested. Under such circumstances, a thrombolytic agent having thrombolyzing activity when administered in a small dose and having only a low level of side effects such as causing a gastro-internal hemorrhage has been eagerly sought.

In recent years, a plasminogen activator separated and purified from a tissue cultured liquor of human melanoma cells has been proposed to serve similar purposes (see Japanese Patent Application (OPI] No. 28009/82 (the term "OPI" as used herein refers to a "published unexamined Japanese patent application")). However, since tumor cells are used as a starting material and these are problems with antigenicity and carcinogenicity, such a plasminogen activator cannot be presented for practical use.

The present inventors have discovered, as a result of their study on various tissue cultured liquors of human normal tissue derived cells, that a substance having plasminogen activator activity different from urokinase is contained therein, and they have successfully separated and purified it (see Japanese Patent Application (OPI) No. 51220/84).

This plasminogen activator is separated from a tissue cultured liquor of normal tissue derived cells and thus does not have the above-described drawbacks of the plasminogen activator derived from melanoma cells.

Thereafter, as a result of the present inventors' investigations on this substance having the activity of plasminogen activator, it has been found that the substance consists of a plurality of proteins, that the substance has protein fragments consisting of a single-chain and a double-chain and that both of the protein fragment of a single-chain structure and that of a double-chain structure have the activity of plasminogen activator.

In addition tissue plasminogen activators are known from Journal of Biological Chemistry, Vol. 256, 1981, pp. 7035 - 7041; Journal of Biological Chemistry, Vol. 255, 1980, pp. 3665 - 3672; WO-A-83/03101 and EP-A-99126

Accordingly, an object of this invention is to provided a novel process for the production of a purified double-chain plasminogen activator, having high affinity to fibrin and exhibiting a thrombolytic effect when administered in a small dose.

This invention provides a process for the production of a double-chain plasminogen activator I obtained from a tissue cultured liquor of human normal tissue derived cells via a conversion of a single-chain plasminogen activator II, said plasminogen activators having the following properties:

a) molecular weight measured by gel filtration:
63,000 ± 10,000,
b) isoelectric point: 7.0 - 8.5,
c) affinity to fibrin: present,
d) affinity to concanavalin A: present,
e) optimum pH: 7 - 9.5,
f) performance on reduction treatment: (II) single-chain structure: substantially does not decompose; (I) double-chain structure: decomposes, and
g) hydrolysis activity on synthetic substrates:

|  | Hydrolysis Activity(%) [1] | |
| Synthetic Substrate | Single-Chain Structure | Double-Chain Structure |
| --- | --- | --- |
| Pro-Phe-Arg-MCA | − | + |
| Z-Phe-Arg-MCA | − | − |
| Boc-Glu-Lys-Lys-MCA | − | − |
| Boc-Phe-Ser-Arg-MCA | + | ++++ |
| Glt-Gly-Arg-MCA | − | ++ |
| Boc-Ileu-Glu-Gly-Arg-MCA | + | +++ |
| Boc-Val-Pro-Arg-MCA | + | + |
| Suc-Leu-Leu-Val-Thr-MCA | − | − |
| Boc-Leu-Ser-Thr-Arg-MCA | − | + |
| Boc-Leu-Thr-Arg-MCA | + | ++ |
| Suc-Ala-Ala-Pro-Phe-MCA | − | − |
| Boc-Val-Leu-Lys-MCA | − | + |
| Boc-Leu-Gly-Arg-MCA | + | ++++ |

[1] Indicated in the following five-rating evaluation, calculated based on 1 μM aminomethylcoumarin as 100%.

−: <2%

+: 2 − 30%

++: 31 − 60%

+++: 61 − 90%

++++: >90%

## BRIEF DESCRIPTION OF THE DRAWINGS

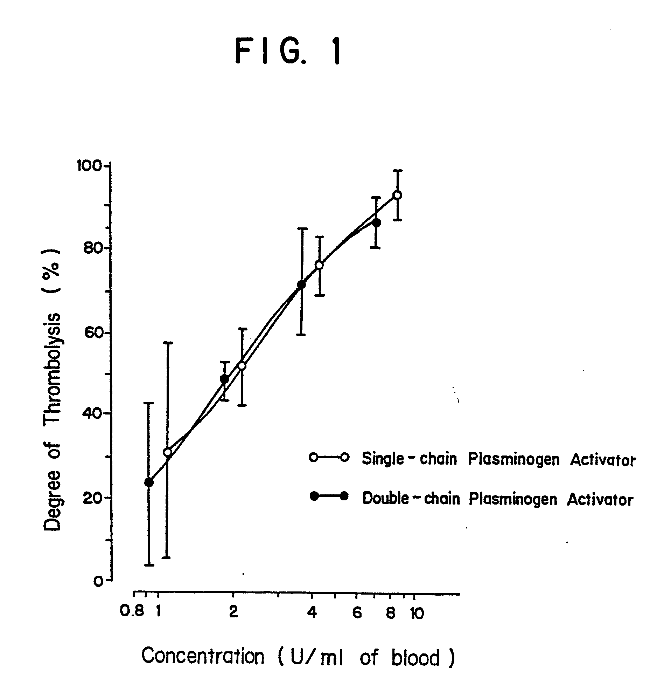

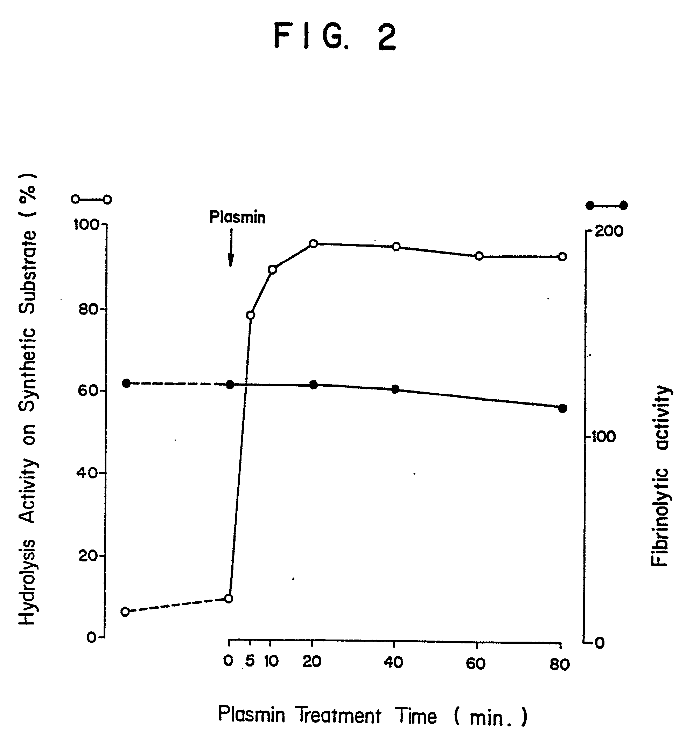

Figure 1 is a graph plotting the concentration against degree of thrombolysis for the single-chain plasminogen activator and the double-chain plasminogen activator of this invention and Figure 2 is a graph showing the amount of double-chain plasminogen activator produced from the relation of a period of time for the plasmin treatment of a single-chain plasminogen activator with the hydrolysis activity on a synthetic substrate and the fibrinolytic activity.

The single-chain plasminogen activator can be selectively produced by culturing human normal tissue derived cells such as cells derived from human embryonic kidney, intestines, lungs, heart, ureter, skin or foreskin, or the whole embryo, human placenta derived cells or cells derived from human kidney, intestines, lungs, thyroid gland, heart, ureter or skin, in an appropriate growth medium in the presence of a proteinase inhibitor and separating and purifying

EP 0 151 996 B1

it from the tissue cultured liquor in the presence of a proteinase inhibitor.

The double-chain plasminogen activator can be produced by collecting a fraction having the activity of plasminogen activator obtained from a tissue cultured liquor of human normal tissue derived cells as described above and subjecting it to an enzyme treatment or incubating it at a temperature of about 10°C to about 80°C for about 5 days to about 10 days in any steps of separating and purifying it.

The tissue cultured liquor of human normal tissue derived cells employed in this invention may be any of those obtained by cultivating cells capable of producing a plasminogen activator in various suitable culture media, and examples thereof include such culture media as those described in Japanese Patent Application (OPI) Nos. 107510/79, 107511/79, 19001/80 and 139323/80, and Japanese Patent Publication No. 5159/82.

More specifically, a plasminogen activator can be produced by contacting the cells which are proliferated in a conventional manner for cultivation of animal cells (e.g., as described in Tissue Culture Methods and Applications , edited by P.F. Kruse et al., Academic Press, New York, San Francisco, London (1973)) with a nutrient solution containing carbon sources, nitrogen sources and optionally inorganic salts and/or other additives such as amino acids, vitamins, peptides, hormones, saccharides and organic acids. Usually, the production of a plasminogen activator is performed using at least 0.2 mℓ of the nutrient solution per 100,000 cells at a temperature of from 25 to 40°C, preferably from 30 to 38°C. During the production, the pH of the nutrient solution is adjusted to 6 to 8, preferably 6.8 to 7.6. The period required for the production is usually 4 to 30 days, but may exceed 30 days. Since the speed of production gradually decreases in the later stage of production, the period which provides the best efficiency is chosen for commercial production. Representative cultivating methods are illustrated in Reference Examples 1 to 4 described hereinbelow.

The overall method for separating and purifying the plasminogen activator from the cultured liquor involves steps conventionally employed in protein chemistry, for example, adsorption using carriers, ion exchange, fractional precipitation, gel filtration, electrophoresis, various types of affinity chromatography, especially those using specific antibodies, etc. They may be employed either alone or in combination.

The proteinase inhibitors which can be used in the steps of tissue culturing human normal tissue derived cells, separating a plasminogen activator from a tissue cultured liquor and purifying it for the selective production of a single-chain plasminogen activator include, for example, natural proteinase inhibitors such as aprotinin, soybean trypsin inhibitor and leupeptin, and synthetic inhibitors such as FOY (product of Ono Pharmaceutical Co., Ltd.), ε-aminocaproic acid, trans-4-aminomethylcyclohexanecarboxylic acid. These proteinase inhibitors may be employed either alone or in combination. The amount of the proteinase inhibitors to be employed varies depending on the proteinase chosen. For example, the amount of aprotinin employed is typically from 1 KIU/mℓ to 1,000 KIU/mℓ, the amount of soybean trypsin inhibitor is typically from 100 ng/mℓ to 1 mg/mℓ and the amount of leupeptin is typically from $10^{-3}$ M to $10^{-1}$ M.

One example of a specific overall method for the selective production of the single-chain plasminogen activator involves tissue culturing human normal tissue derived cells in the presence of aprotinin as the proteinase inhibitor, adding ammonium sulfate to the obtained tissue cultured liquor or its concentrated cultured liquor in the presence or absence of aprotinin, separating the formed precipitates, dissolving them in an ammonium thiocyanate solution containing sodium chloride and aprotinin, passing the solution through an anti-urokinase Ig-G Sepharose® column and adsorbing the solution onto a fibrin Sepharose® column. Thereafter, an eluate obtained by using arginine containing aprotinin as an eluent is further passed through an anti-urokinase Ig-G Sepharose® column, and freeze dried. The thus-obtained powder is dissolved in water and is then gel filtered using Sephadex® G-150 (registered trademark by Pharmacia Co.) to obtain the intended single-chain plasminogen activator.

The selective production of the double-chain plasminogen activator can be effected by converting a mixture of a single-chain plasminogen activator and a double-chain plasminogen activator obtained from a tissue cultured liquor of human normal tissue derived cells or the single-chain plasminogen activator as obtained above into a double-chain plasminogen activator. This conversion can be effected by subjecting a fraction containing the plasminogen activator from a tissue cultured liquor to an enzyme treatment or by incubating the fraction at about 10°C to about 80°C, preferably at about 20°C to about 40°C, for about 5 days to about 10 days in any step of separating and purifying a plasminogen activator. Also the conversion can be effected by subjecting the purified single-chain plasminogen activator as obtained above to an enzyme treatment. The enzyme treatment is typically carried out at a temperature of about 10°C to about 45°C, preferably at a temperature of about 25°C to about 40°C, and the period of time for the enzyme treatment is typically about 5 minutes to about 3 hours.

The enzyme treatment can be carried out by any methods employed in conventional enzyme reactions

4

and includes contacting the mixture of a single-chain plasminogen activator and a double-chain plasminogen activator or the single-chain plasminogen activator with an enzyme directly or with an immobilized enzyme.

The enzymes which can be employed in this invention include, for example, plasmin, kallikrein, trypsin, activated Factor XI and Factor XII of coagulation. The amount of the enzymes to be employed varies depending on the enzyme selected. For example, the amount of plasmin employed is typically from 0.001 CU/ml to 10 CU/ml, that of kallikrein is typically from 1 KU/ml to 1,000 KU/ml and that of trypsin is typically from 2 ng/ml to 20 μg/ml. As the carrier for immobilizing an enzyme there can be employed any conventional carriers used for immobilizing an enzyme such as Sepharose.

One example of a specific overall method for the production of the double-chain plasminogen activator involves passing a tissue cultured liquor of human normal tissue derived cells or its concentrated cultured liquor through a plasmin Sepharose® column, adding ammonium sulfate to the solution obtained, separating the formed precipitates, dissolving them in an ammonium thiocyanate solution containing sodium chloride, passing the solution through an anti-urokinase Ig-G Sepharose® column and adsorbing the solution onto a fibrin Sepharose® column. Thereafter, an elute obtained by using arginine as an eluent is further passed through an anti-urokinase Ig-G Sepharose® column and freeze dried. The thus-obtained powder is dissolved in water and then gel filtered using Sephadex® to obtain the intended double-chain plasminogen activator.

There is no doubt that the thus-obtained single-chain plasminogen activator and double-chain plasminogen activator are plasminogen activators because they do not dissolve plasminogen-free fibrin but dissolve plasminogen-containing fibrin.

The physical and chemical properties of the single-chain plasminogen activator and the double-chain plasminogen activator thus obtained are described below. The titer measurement (fibrinolytic activity) was carried out by the following procedures (this also applies to the experiments described herein below).

Using an agar fibrin-added plate prepared by using 95% clcttable fibrinogen (plasminogen content: about 50 casein units/g clottable protein) as a starting material, the measurement was carried out by a plate method employing urokinase as the standard. A solution of the substances of this invention was diluted with a 0.067 M tris-HCl buffer (pH 8.0) containing 1% gelatine, 0.1 M sodium chloride and 0.1% sodium azide, and the concentration of the substances of this invention exhibiting the lyzing zone same as that of 10 IU/ml of urokinase on the fibrin plate was designated as 10 U/ml.

a) Molecular Weight: 63,000 ± 10,000

This was measured by gel filtration using Sephadex® G-150 equilibrated with a 0.01 M phosphate buffer (pH 7.0) containing 1.5 M sodium chloride, 0.1 M EDTA, 0.1 M arginine and 0.1% Tween 80 (registered trademark by Kao Atlas). When measured by SDS (sodium dodecyl sulfate) electrophoresis (non-reduction), it was about 70,000.

b) Isoelectric Point: 7.0 - 8.5

This was measured by isoelectric point electrophoresis using an ampholyte at about 0°C.

c) Affinity to Fibrin:

20 μl of each of the substances of this invention (500 U/ml) was added to 950 μl of physiological saline containing 0.2% of plasminogen-free fibrinogen, and then 50 μl of thrombin (30 U/ml) was added thereto. The resulting solution was allowed to stand for 1 hour at room temperature, whereby fibrin was formed. The fibrin was separated, dehydrated and washed with physiological saline. By extracting the substances of this invention from the fibrin with 1 ml of a 2 M ammonium thiocyanate solution, it was found that about 70% of the substances was incorporated in the fibrin. For comparison, when urokinase (500 IU/ml) was used, it was not incorporated in the fibrin at all.

d) Affinity to Concanavalin A:

When 2 ml of each of the substances of this invention (30 U/ml) was dissolved in physiological saline, adsorbed onto a column (0.5 x 4 cm) of concanavalin A Sepharose® (manufactured by Pharmacia Co.), and washed with a 1 M sodium chloride solution, nearly 100% of the substance was adsorbed.

e) Optimum pH: 7 - 9.5

To 50 μl of each of the substances of this invention dissolved in physiological saline were added 50 μl of plasminogen (8 CU/ml) dissolved in physiological saline containing 10% glycerine and 100 μl of one of a 0.05 M citrate buffer (pH 5.0 or 6.0) containing 0.10 M sodium chloride, a phosphate buffer (pH 6.0, 7.0 or 8.0) or a glycine-sodium hydroxide buffer (pH 8.0, 9.0, 10.0 or 11.0) (i.e., seven buffers, each at a different pH of 5.0, 6.0, 7.0, 8.0, 9.0, 10.0 or 11.0), and each was preincubated at 37°C for 30 minutes. Thereafter, 500 μl of Boc-Glu-Lys-Lys-MCA (Peptide Institute, Inc.) dissolved in a 0.15 M tris-HCl buffer (pH 8.0) was added, and after incubation at 37°C for 15 minutes, the reaction was terminated by adding 1 ml of acetic acid, and the formed aminomethylcoumarin was measured by fluorometry to determine the optimum pH.

f) Stability:

(1) Single-Chain Plasminogen Activator

A phosphate buffer containing the substance of this invention and having a pH of 7.0 was prepared and then a virulyzing treatment at 100°C for 1 minute was carried out and deactivation by about 15% was observed.

(2) Double-Chain Plasminogen Activator

The double-chain plasminogen activator dissolved in physiological saline (100 U/mℓ) containing 0.02% Tween® 80 was subjected to heat treatment at 55°C for 3 hours. A reduction in activity was not observed.

g) Hydrolysis Activity on Various Synthetic Substrates:

To 50 μℓ of each of the substances of this invention (100 U/mℓ) was added 0.1 mM of each of various substrates dissolved in 450 μℓ of a 0.05 M tris-HCℓ buffer (pH 8.0) containing 0.1 M sodium chloride, and each reaction was effected at 37°C for 15 minutes. The reaction was terminated by adding 0.5 mℓ of 20% acetic acid, and then measurement for formed aminomethylcoumarin was carried out at an excitation wavelength of 370 nm, a slit width of 5 nm, an emission wavelength of 460 nm and a slit width of 5 nm to determine the hydrolysis activity.

The results are shown in Table 1.

## TABLE 1

| Synthetic Substrate[2, 3] | Hydrolysis Activity (%)[1] | |
|---|---|---|
| | Single-Chain Structure | Double-Chain Structure |
| Pro-Phe-Arg-MCA | − | + |
| Z-Phe-Arg-MCA | − | − |
| Boc-Glu-Lys-Lys-MCA | − | − |
| Boc-Phe-Ser-Arg-MCA | + | ++++ |

(cont'd)

| Synthetic Substrate[2, 3] | Hydrolysis Activity (%)[1] | |
| --- | --- | --- |
| | Single-Chain Structure | Double-Chain Structure |
| Glt-Gly-Arg-MCA | − | ++ |
| Boc-Ileu-Glu-Gly-Arg-MCA | + | +++ |
| Boc-Val-Pro-Arg-MCA | + | + |
| Suc-Leu-Leu-Val-Thr-MCA | − | − |
| Boc-Leu-Ser-Thr-Arg-MCA | − | + |
| Boc-Leu-Thr-Arg-MCA | + | ++ |
| Suc-Ala-Ala-Pro-Phe-MCA | − | − |
| Boc-Val-Leu-Lys-MCA | − | + |
| Boc-Leu-Gly-Arg-MCA | + | ++++ |

(Note)

[1] Hydrolysis activity is indicated in five-rating evaluation as shown below, in terms of average concentration (measured five times) of the hydrolyzed product, aminomethylcoumarin, calculated based on 1 µM aminomethylcoumarin as 100%.

−: <2% (below lower limit of detection)

+: 2 − 30%

++: 31 − 60%

+++: 61 − 90%

++++: >90%

7

*2  These substrates are described in S. Iwanaga et al., <u>Biochemistry, Pathophysiology, and Clinical Aspects</u>, edited by S. Fujii et al., Plenum Publishing Co., 1979, p. 147.

*3  The -MCA (-methylcoumarinamide) of substrates is released in the form of aminomethylcoumarin upon hydrolysis.

h) Influence of Various Proteinase Inhibitors:

To 50 $\mu l$ of either each of the substances (100 U/m$l$) or urokinase (100 IU/m$l$) were added 50 $\mu l$ of one of a number of solutions of various proteinase inhibitors and 300 $\mu l$ of a 0.05 M tris-HC$l$ buffer (pH 8.0) containing 0.1 M sodium chloride, and each reaction was effected at 37°C for 5 minutes. Thereafter, 100 $\mu l$ of 0.1 mM of Boc-Phe-Ser-Arg-MCA (Peptide Institute, Inc.) was added, and each reaction was effected at 37°C for 60 minutes. Thereafter, 0.5 m$l$ of 20% acetic acid was added to terminate the reaction which was then measured for the formed aminomethylcoumarin at an excitation wavelength of 370 nm, a slit width of 2 nm, an emission wavelength of 460 nm and a slit width of 2 nm to determine the hydrolysis activity.

The concentrations (IC$_{50}$) of the proteinase inhibitors which inhibit 50% of the activity of the substances of this invention were determined and the results are shown in Table 2.

<u>TABLE  2</u>

| | IC$_{50}$ (M) | |
|---|---|---|
| Inhibitor | Single-Chain Structure | Double-Chain Structure |
| Aprotinin | >500 [*4] | >500 [*4] |
| FOY [*5] | $1.7 \times 10^{-5}$ | $9.2 \times 10^{-5}$ |
| Pepstatin | $10^{-3}$ | $10^{-3}$ |
| Leupeptin | $2.8 \times 10^{-4}$ | $5.7 \times 10^{-4}$ |
| Chymostatin | $10^{-3}$ | $10^{-3}$ |

(Note)

*4  KIU

*5  Ono Pharmaceutical Co., Ltd.

i) Performance on Reduction Treatment

Each of the substances (0.5 mg protein/m$l$) was mixed with a tris-HC$l$ buffer (pH 6.8) containing 2% sodium dodecyl sulfate, 2% $\beta$-mercaptoethanol and 40% glycerine at a volume ratio of 1:1 and the mixed solution was subjected to heat treatment at 100°C for 5 minutes and then to SDS (sodium dodecyl sulfate)-polyacrylamide gel electrophoresis according to the method of Laemmli et al. (Nature , 227 , 680, 1970).

The single-chain plasminogen activator was confirmed by a single band at a molecular weight of about 68,000 and the double-chain plasminogen activator of this invention was confirmed by two bands at a molecular weight of about 33,000 and about 35,000, respectively. The molecular weight was identified using a calibration kit (manufactured by Pharmacia Fine Chemicals Co.).

When the plasminogen activator produced without use of any proteinase inhibitor in the steps of culturing, separation and purification was subjected to the reduction treatment, it showed three bands at a molecular weight of about 33,000, about 35,000 and 68,000, respectively.

j) Enhancement of Activity with Fibrin:

To 200 $\mu\ell$ of a 0.05 M tris-HC$\ell$ buffer (pH 7.5) containing 0.05% of fibrinogen and 0.1 M sodium chloride were added subsequently 50 $\mu\ell$ of a plasminogen solution (10 CU/m$\ell$), 50 $\mu\ell$ of either the substances of this invention (5 U/m$\ell$) or urokinase (5 IU/m$\ell$), 100 $\mu\ell$ of a 0.1 mM Boc-Val-Leu-Lys-MCA solution and 100 $\mu\ell$ of a thrombin solution (2 U/m$\ell$), and the reaction was carried out at 20°C for 1 hour. The reaction was terminated by adding 500 $\mu\ell$ of 20% acetic acid, and then hydrolysis activity was measured in the same manner as in the test (g) above. Further, the same procedure as above was repeated except fibrinogen was not added. The results are shown in Table 3.

## TABLE 3

| | Hydrolysis Activity (%) [*6] | | |
| --- | --- | --- | --- |
| Fibrinogen | Single-Chain Structure (5 U/m$\ell$) | Double-Chain Structure (5 U/m$\ell$) | Urokinase (5 IU/m$\ell$) |
| Added | 69.1 | 70.4 | 5.9 |
| Not added | 7.5 | 8.0 | 28 |

(Note)

   *6   Calculated based on 10 µM aminomethylcoumarin as 100%.

As evident from the various properties described above, the single-chain plasminogen activator and the double-chain plasminogen activator are purified substances different from urokinase derived from human urine or a tissue cultured liquor of kidney cells.

Thereafter, the thrombolytic activity of the single-chain plasminogen activator and the double-chain plasminogen activator of this invention was measured by the Chandler's loop method (Quart. J. Exp. Physiol. , 46 , 1 (1961)). The blood used was human fresh blood, the thrombus forming time was 30 minutes, and the thrombolysis time was 4 hours.

As a result, it was confirmed that the thrombolytic activity of the single-chain plasminogen activator and the double chain plasminogen activator was 30 times as strong as that of urokinase. Therefore, the plasminogen activators are extremely useful as a thrombolytic agent which provides a strong thrombolytic effect upon administration of a small dose.

The substances are preferably administered intravenously, and the dose, although varying depending on the condition of the patient, may be in the range of 50 to 1,000,000 units per day. The method for intravenous administration is preferably by injection, or it may be administered by dissolving in a transfusion medium, etc.

The substances can be formulated into, e.g., an injectable preparation, for example, by mixing the substance with a conventional excipient for injection, a buffer, an isotonic agent, a filler, a stabilizer or the like, dissolving the mixture in distilled water for injection, and freeze-drying and/or vacuum-drying the solution to obtain a drug composition which is filled in a vial for injection.

Other applications of the plasminogen activator, in addition to medical use as a thrombolytic agent, are for preventing the formation of a thrombus by, for example, combining it with materials such as artificial blood vessels, artificial organs, etc., or as a diagnostic agent for thrombosis, etc.

### REFERENCE EXAMPLE 1

Human embryonic kidney cells were implanted in a 100 mm plastic dish at a density of $7 \times 10^4$ cells/m$\ell$, and subjected to sufficient proliferation using 10 m$\ell$ of Medium MEM (minimum essential medium obtained from Eagle) containing 10% fetal calf serum as a growth medium at 37°C in air containing 5% carbon dioxide. After 5 days, the cells were washed with physiological saline, and the medium was replaced by 20 m$\ell$ of a serum-free producing medium consisting of Medium 199 (obtained from Morgan) containing 0.5% lactalbumin hydrolysate and 0.8% fumaric acid. After maintaining the cells in Medium 199 for 7 days, the medium was replaced by a fresh producing medium as described above, and the cultured liquor containing the substance of this invention was recovered.

### REFERENCE EXAMPLE 2

Human embryonic lung cells were implanted to a 500 m$\ell$ spinner flask at a density of $10^5$ cells/m$\ell$ together with Cytodex I (bead carrier for cell culture, registered trademark by Pharmacia Co.) at a concentration of 2.5 mg/m$\ell$, and suspension cultured by using 300 m$\ell$ of Medium MEM containing 10% fetal calf serum as a growth medium at 37°C in air containing 5% carbon dioxide, while stirring at a rotation of 60 rpm. After sufficient proliferation of the cells by their cultivation for 8 days, the bead carrier to which the cells had been adhered was washed with physiological saline, and the medium was replaced by 300 m$\ell$ of serum-free Medium 199 containing 0.5% lactalbumin hydrolysate and cultivation was continued for 25 days with stirring at a rotation of 60 rpm, while the medium was replaced on every fifth day. Thus, the cultured liquor containing the substance of this invention was recovered.

### REFERENCE EXAMPLE 3

The procedure of Reference Example 1 was repeated except that Medium 199 containing 0.5% lactalbumin hydrolysate, 0.8% fumaric acid and 20 KIU/m$\ell$ aprotinin was used instead of the Medium 199, and a cultured liquor containing the substance of this invention was obtained.

### REFERENCE EXAMPLE 4

The procedure of Reference Example 2 was repeated except that Medium 199 containing 0.5% lactalbumin hydrolysate and 20 KIU/m$\ell$ aprotinin was used instead of the Medium 199, and a cultured liquor containing the substance was obtained.

Production of Single-Chain Plasminogen Activator

### REFERENCE-EXAMPLE 5

Ammonium sulfate was added to 32 $\ell$ of a human embryonic kidney tissue cultured liquor as obtained by Reference Example 1 in a proportion of 300 g/$\ell$, and stirred at 0°C for 2 hours. The formed precipitates were collected by centrifugation at 5,000 rpm for 30 minutes and dissolved in a 0.02 M tris-HC$\ell$ buffer (pH 7.5) containing 0.5 M sodium chloride, 0.1% Tween® 80 and 25 KIU/m$\ell$ aprotinin, and the resulting solution was dialyzed against a solution having the same composition as the above-described solution at 4°C overnight. The obtained solution containing the substance of this invention was 320 m$\ell$ in liquid volume, and the activity of the solution was 18 U/m$\ell$. Further, the specific activity of the solution (activity per unit weight of the proteins (including the substance of this invention) contained therein) was measured according to the method described in White W.F., Biochemistry , 5 (1966) wherein concentration of the proteins in the solution was measured from their absorbance at 280 nm. As a result, the specific activity was 9.1 U/A280. This solution was adsorbed onto a zinc Sepharose® column (4Φ x 7.1 cm) and then eluted with a 0.02 M tris-HCℓ buffer (pH 7.5) containing 50 mM imidazole, 0.5 M sodium chloride, 0.1% Tween® 80 and 25 KIU/mℓ aprotinin. The elute was 118 mℓ in liquid volume and the activity of the elute was 41 U/mℓ and its specific activity was 320 U/A280.

This elute was dialyzed against physiological saline containing 0.1% Tween® 80 and 25 KIU/mℓ aprotinin, passed through an anti-urokinase Ig-G Sepharose® column and continuously adsorbed onto an arginine Sepharose® column (1.5Φ x 8 cm). After sufficient washing with a 0.5 M sodium chloride solution containing 0.1% Tween 80 and 25 KIU/mℓ aprotinin, a 0.5 M arginine solution containing 0.1% Tween 80 and 25 KIU/mℓ aprotinin was used to elute the substance of this invention. The obtained solution had a liquid volume of 47 mℓ, and the activity was 87 U/mℓ and its specific activity was 3,500 U/A280.

The above-obtained solution was concentrated by ultrafiltration and gel filtered through a column (1.5Φ x 100 cm) of Sephadex® G-150 equilibrated with a 0.01 M phosphate buffer (pH 7.0) containing 1.5 M sodium chloride and 0.1% Tween 80 to collect a fraction having activity. The obtained solution containing the plasminogen activator was 15 mℓ in liquid volume, and the activity was 210 U/mℓ and its specific activity was 14,000 U/A280.

According to the analysis of the above-obtained substance by SDS-polyacrylamide electrophoresis, it was not decomposed by the reduction treatment in the presence of a buffer containing 1% dodecyl sodium sulfate, 1% β-mercaptoethanol and 20% glycerine at 100° C for 5 minutes, and thus it was confirmed as the single-chain structure.

REFERENCE-EXAMPLE 6

1.5 ℓ of a human embryonic lung tissue cultured liquor as obtained by each of Reference Examples 2 and 4 was passed through an anti-urokinase Ig-G Sepharose® column, and then adsorbed onto a fibrin Sepharose® column (1.5Φ x 12 cm). After washing sufficiently with a 0.5 M sodium chloride solution containing 0.1% Tween® 80 and 25 KIU/mℓ aprotinin, a 0.5 M arginine solution containing 0.1% Tween 80 and 25 KIU/mℓ aprotinin was used to elute and collect 65 mℓ of a fraction containing the activity of the substance of this invention. The activity of this solution was 68 U/mℓ and the specific activity was 1,020 U/A280. This solution was dialyzed against physiological saline containing 0.1% Tween 80 and 25 KIU/mℓ aprotinin, then adsorbed onto a concanavalin A Sepharose® column (1Φ x 25 cm), and washed with a 0.01 M phosphate buffer (pH 7.0) containing 1 M sodium chloride, 0.1% Tween® 80 and 25 KIU/mℓ aprotinin, followed by eluting the substance of this invention according to a linearly gradient elution method using the above buffer at an initial stage while continuously changing the composition of buffer to a 0.01 M phosphate buffer (pH - 7.0) containing 0.4 M methylmannoside, 2 M ammonium thiocyanate, 0.1% Tween 80 and 25 KIU/mℓ aprotinin. The obtained solution was 31 mℓ in liquid volume, and the activity was 102 U/mℓ and its specific activity was 6,500 U/A280. After dialysis, the dialysate was concentrated by ultrafiltration and gel filtered by Sephadex® G-150 to recover a 15 mℓ fraction containing activity. The activity was 161 U/mℓ and the specific activity was 13,100 U/A280.

According to the analysis of the above-obtained substance of this invention SDS-polyacrylamide electrophoresis, it was not decomposed by the reduction treatment in the presence of a buffer containing 1% dodecyl sodium sulfate, 1% β-mercaptoethanol and 20% glycerine at 100° C for 5 minutes, and thus, it was confirmed to be of a single-chain structure.

REFERENCE-EXAMPLE 7

From 3.4 ℓ of a human embryonic foreskin tissue cultured liquor obtained in the same manner as in Reference Example 3 were removed insolubles by centrifu galization and the obtained solution was adsorbed onto a zinc Sepharose® column (4Φ x 7.5 cm) equilibrated with a 0.02 M tris-HCℓ buffer (pH 7.5) containing 0.1 M sodium chloride, 0.1% Tween® 80 and 25 KIU/mℓ aprotinin, then washed well with a solution having the same composition as the buffer used for equilibration, and eluted with a 0.05 M tris-HCℓ buffer (pH 7.5) containing 50 mM imidazole, 0.5 M sodium chloride, 0.1% Tween® 80 and 25 KIU/mℓ aprotinin. The obtained solution had a liquid volume of 160 mℓ, and the activity was 80 U/mℓ and its specific activity was 236 U/A280.

This solution was adsorbed onto a Sepharose 4B column combined with a specific antibody Ig-G to the plasminogen activator obtained from a rabbit, then washed well with physiological saline, and eluted with a 0.2 M glycine-HCℓ buffer (pH 2.5) containing 25 KIU/mℓ aprotinin. The pH of the elute was immediately raised to near neutrality and its activity was measured, wherdby a solution having a liquid volume of 200 mℓ, an activity of 116 U/mℓ and a specific activity of 4,800 U/A280 was obtained. This solution was concentrated by ultrafiltration and subjected to the gel-filtration using a Sephacryl® S-200 column (1.5Φ x 100 cm) equilibrated with a 0.02 M phosphate buffer containing 0.1% Tween® 80 and 1 M sodium chloride to recover a fraction containing activity. The obtained solution was 15 mℓ and had an activity of 4,800 U/mℓ and a specific activity of 16,000 U/A280.

According to the analysis of the above-obtained substance of this invention by SDS-polyacrylamide electrophoresis, it was not decomposed by the reduction treatment in the presence of a buffer containing 1% dodecyl sodium sulfate, 1% $\beta$-mercaptoethanol and 20% glycerine at 100°C for 5 minutes, and thus, it was confirmed to be of a single-chain structure.

Production of Double-Chain Plasminogen Activator :

EXAMPLE 4

The single-chain plasminogen activator separated and purified in Example 1 was dissolved in a 0.01 M phosphate buffer (pH 7.4) containing 1% human serum albumin and 0.14 M sodium chloride to prepare a 250 U/mℓ plasminogen activator solution. To 200 μℓ of this solution were added 300 μℓ of a plasmin solution *7 and the reaction was effected at 37°C and terminated after a predetermined period of time by neutralizing the plasmin activity with 200 μℓ of an aprotinin buffer added.

By using 70 μℓ of the obtained solution was determined the hydrolysis activity of 0.1 mM of the synthetic substrate Boc-Phe-Ser-Arg-MCA *8. The results of the titer measurement of the substances of this invention before and after the plasmin treatment are shown in Figure 2. The single-chain plasminogen activator was 100% converted into the double-chain plasminogen activator by this plasmin treatment and a reduction in the fibrinolytic activity was not observed before and after the plasmin treatment.

EXAMPLE 5

A mixed solution of a single-chain plasminogen activator and a double-chain plasminogen activator (3,000 U/mℓ) obtained by concentrating a human embryonic kidney tissue cultured liquor as obtained by each of Reference Examples 1 and 2 was dissolved in a 0.02 M tris-HCℓ buffer containing 0.15 M sodium chloride. 2 mℓ of the obtained solution was mixed with 1 mℓ of plasmin Sepharose®*9 and the reaction was effected at 25°C for 5 hours. After the reaction, the plasmin Sepharose® was separated by filtration and the content of the double-chain plasminogen activator was determined from the results of measurement of the hydrolysis activity on the synthetic substrate and the reduction treatment which was carried out in the same manner as in Example 4. As a result, it was confirmed that the single-chain plasminogen activator was

*7 The plasmin solution was prepared by passing a commercially available plasmin solution (product of Green Cross Corp.) through an anti-urokinase Ig-G Sepharose® column and a lysine Sepharose® column to give a urokinase-free plasmin solution and diluting the plasmin solution with the 0.01 M phosphate buffer (pH 7.4) containing 1% human serum albumin and 0.14 M sodium chloride to give a 0.04 CU/mℓ plasmin solution.

*8 The synthetic substrate Boc-Phe-Ser-Arg-MCA is hardly hydrolyzed by the single-chain plasminogen activator.

*9 The plasmin Sepharose® was prepared by mixing 1 mℓ of human plasminogen (100 CU/mℓ) and 0.1 mℓ of urokinase Sepharose® (100 UI/mℓ), allowing the mixture to react at room temperature overnight resulting in plasmin, separating the urokinase Sepharose® from the plasmin solution by filtration, allowing the plasmin solution to react with BrCN-activated Sepharose®. The activity of plasmin was measured using the resultant plasmin Sepharose® and a synthetic substrate Boc-Glu-Lys-Lys-MCA and was found to be about 0.5 CU/mℓ-resin.

100% converted into the double-chain plasminogen activator by the plasmin Sepharose® treatment.

EXAMPLE 6

Ammonium sulfate was added to 3.6 ℓ of a human embryonic foreskin tissue cultured liquor obtained in the same manner as in Reference Example 3 to give a 60% saturation, and stirred at 0°C for 2 hours. The formed precipitates were collected by centrifugation at 5,000 rpm for 30 minutes and dissolved in a 0.02 M tris-HCℓ buffer (pH 7.5) containing 0.15 M sodium chloride and 0.1% Tween® 80, and the resulting solution was dialyzed against a solution having the same composition as the above-described solution at 4°C overnight and then incubated at 25°C for 1 week. This solution was adsorbed onto a zinc Sepharose® column (2.6Φ x 6 cm) and then eluted with a 0.05 M tris-HCℓ buffer (pH 7.5) containing 50 mM imidazole, 0.5 M sodium chloride and 0.1% Tween® 80. The elute was 156 mℓ in liquid volume and the activity was 173 U/mℓ and its specific activity was 850 U/A280.

This elute was diluted with a 0.02 M tris-HCℓ buffer (pH 7.6) containing 0.1% Tween® by ten times and then adsorbed onto a lysine Sepharose® column (1.6Φ x 6 cm) equilibrated with a solution having the same composition as the buffer used for dilution and then eluted with a 0.02 M tris-HCℓ buffer (pH 7.6) containing 0.05 M sodium chloride, 0.5 M potassium thiocyanate, 0.1 M ε-amino-n-caproic acid and 0.1% Tween® 80. The obtained solution had a liquid volume of 20.5 mℓ, and the activity was 103 U/mℓ and its specific activity was 21,000 U/A280.

This solution was subjected to the reduction treatment and the SDS-polyacrylamide electrophoresis as described in i) Performance on reduction treatment and as a result, it was found that the single-chain plasminogen activator was almost 100% converted into the double-chain plasminogen activator.

EXAMPLE 7

The procedure of Example 6 was repeated except that a mixed solution of the single-chain plasminogen activator and the double-chain plasminogen activator (3,000 U/mℓ) obtained by concentrating a human embryonic lung tissue cultured liquor as obtained in Reference Example 2 was employed instead of that obtained by concentrating a human embryonic kidney tissue cultured liquor as obtained by Reference Example 2.

As a result, it was confirmed that the single-chain plasminogen activator was 100% converted into the double-chain plasminogen activator by the plasmin Sepharose® treatment.

## Claims

1. A process for the production of a double-chain plasminogen activator I, which comprises subjecting a fraction containing the plasminogen activator from a tissue cultured liquor of human normal tissue derived cells to an enzyme treatment in its separation and purification step or subjecting a purified single-chain plasminogen activator II to an enzyme treatment, wherein the plasminogen activators I and II have the following properties:

   I

   a) molecular weight measured by gel filtration:
   63,000 ± 10,000,
   b) isoelectric point: 7.0 - 8.5,
   c) affinity to fibrin: present,
   d) affinity to concanavalin A: present,
   e) optimum pH: 7 - 9.5,
   f) performance on reduction treatment: decomposes,
   and
   g) hydrolysis activity on synthetic substrates as shown below

| Synthetic Substrate | Hydrolysis Activity (%) [1] |
|---|---|
| Pro-Phe-Arg-MCA | + |
| Z-Phe-Arg-MCA | − |
| Boc-Glu-Lys-Lys-MCA | − |
| Boc-Phe-Ser-Arg-MCA | ++++ |

| Synthetic Substrate | Hydrolysis Activity (%) [1] |
|---|---|
| Glt-Gly-Arg-MCA | ++ |
| Boc-Ileu-Glu-Gly-Arg-MCA | +++ |
| Boc-Val-Pro-Arg-MCA | + |
| Suc-Leu-Leu-Val-Thr-MCA | − |
| Boc-Leu-Ser-Thr-Arg-MCA | + |
| Boc-Leu-Thr-Arg-MCA | ++ |
| Suc-Ala-Ala-Pro-Phe-MCA | − |
| Boc-Val-Leu-Lys-MCA | + |
| Boc-Leu-Gly-Arg-MCA | ++++ |

[1] Indicated in the following five-rating evaluation, calculated based on 1 μM aminomethylcoumarin as 100%:

    −:     <2%

    +:    2 − 30%

    ++:   31 − 60%

    +++:  61 − 90%

    ++++:  >90%

II

a) molecular weight measured by gel filtration:
63,000 ± 10,000,
b) isoelectric point: 7.0 - 8.5,
c) affinity to fibrin: present,

d) affinity to concanavalin A: present,

e) optimum pH: 7 - 9.5,

f) performance on reduction treatment: substantially does not decompose, and

g) hydrolysis activity on synthetic substrates:

| Synthetic Substrate | Hydrolysis Activity (%) [1] |
|---|---|
| Pro-Phe-Arg-MCA | - |
| Z-Phe-Arg-MCA | - |
| Boc-Glu-Lys-Lys-MCA | - |
| Boc-Phe-Ser-Arg-MCA | + |
| Glt-Gly-Arg-MCA | - |
| Boc-Ileu-Glu-Gly-Arg-MCA | + |
| Boc-Val-Pro-Arg-MCA | + |
| Suc-Leu-Leu-Val-Thr-MCA | - |
| Boc-Leu-Ser-Thr-Arg-MCA | - |
| Boc-Leu-Thr-Arg-MCA | + |
| Suc-Ala-Ala-Pro-Phe-MCA | - |
| Boc-Val-Leu-Lys-MCA | - |
| Boc-Leu-Gly-Arg-MCA | + |

[1]  Indicated in the following five-rating evaluation,
calculated based on 1 μM aminomethylcoumarin as 100%:

-:   <2%

+:   2 - 30%

2. The process according to Claim 1, wherein the enzyme is plasmin.

3. A process for the production of the double-chain plasminogen activator I as recited in Claim 1, which comprises incubating a fraction containing the plasminogen activator from a tissue cultured liquor of human normal tissue derived cells at a temperature of about 10°C to about 80°C in its separation and purification step.

4. The process according to Claim 3, wherein the temperature is about 20°C to about 40°C.

5. The process according to Claims 3 or 4, wherein the incubating step is carried out for a period of time of about 5 days to about 10 days.

6. The process according to any of the proceeding claims, wherein the human normal tissue derived cells are selected from the group consisting of cells derived from human embryonic kidney, intestines, lungs, heart, ureter, skin or foreskin, or human whole embryo; human placenta derived cells; and cells derived from human kidney, intestines, lungs, thyroid gland, heart, ureter or skin.

**Revendications**

1. Procédé de production d'un activateur de plasminogène I double chaîne, qui consiste à soumettre une fraction contenant l'activateur de plasminogène d'une liqueur mise en culture sur tissu de cellules dérivées de tissu normal humain à un traitement enzymatique dans son étape de séparation et de purification ou à soumettre un activateur de plasminogène II purifié simple chaîne à un traitement enzymatique, où les activateurs de plasminogène I et II ont les propriétés suivantes :

I

a) poids moléculaire mesuré par filtration sur gel : 63.000 ± 10.000,

b) point isoélectrique : 7,0-8,5,

c) affinité à la fibrine : présente,

d) affinité à la concanavaline A : présente,

e) pH optimum : 7-9,5,

f) performance lors d'un traitement de réduction : se décompose et

g) activité à l'hydrolyse sur des substrats de synthèse telle que montrée ci-dessous

| Substrat de synthèse | Activité à l'hydrolyse (%)[*1] |
|---|:---:|
| Pro-Phe-Arg-MCA | + |
| Z-Phe-Arg-MCA | - |
| Boc-Glu-Lys-Lys-MCA | - |
| Boc-Phe-Ser-Arg-MCA | ++++ |
| Glt-Gly-Arg-MCA | ++ |
| Boc-Ileu-Glu-Gly-Arg-MCA | +++ |
| Boc-Val-Pro-Arg-MCA | + |
| Suc-Leu-Leu-Val-Thr-MCA | - |
| Boc-Leu-Ser-Thr-Arg-MCA | + |
| Boc-Leu-Thr-Arg-MCA | ++ |
| Suc-Ala-Ala-Pro-Phe-MCA | - |
| Boc-Val-Leu-Lys-MCA | + |
| Boc-Leu-Gly-Arg-MCA | ++++ |

[*1] Indiquée dans l'évaluation à cinq points qui suit, calculée en se basant sur 1 µM d'aminométhylcoumarine à 100% :

  - : < 2%

  + : 2-30%

  ++ : 31-60%

  +++ : 61-90%

  ++++ : > 90%

II

a) poids moléculaire mesuré par filtration sur gel : 63.000 ± 10.000,

b) point isoélectrique : 7,0-8,5,

c) affinité à la fibrine : présente,

d) affinité à la concanavaline A : présente,

e) pH optimum : 7-9,5,

f) performance lors du traitement de réduction : ne se décompose sensiblement pas, et

g) activité à l'hydrolyse sur des substrats de synthèse :

| Substrat de synthèse | Activité à l'hydrolyse (%)[*1] |
|---|---|
| Pro-Phe-Arg-MCA | − |
| Z-Phe-Arg-MCA | − |
| Boc-Glu-Lys-Lys-MCA | − |
| Boc-Phe-Ser-Arg-MCA | + |
| Glt-Gly-Arg-MCA | − |
| Boc-Ileu-Glu-Gly-Arg-MCA | + |
| Boc-Val-Pro-Arg-MCA | + |
| Suc-Leu-Leu-Val-Thr-MCA | − |
| Boc-Leu-Ser-Thr-Arg-MCA | − |
| Boc-Leu-Thr-Arg-MCA | + |
| Suc-Ala-Ala-Pro-Phe-MCA | − |
| Boc-Val-Leu-Lys-MCA | − |
| Boc-Leu-Gly-Arg-MCA | + |

[*1]   Indiquée dans l'évaluation à cinq points qui suit, calculée en se basant sur 1 $\mu$M d'aminométhylcoumarine à 100% :

－ :  ＜ 2%

＋ :   2-30%

2.  Procédé selon la revendication 1, où l'enzyme est la plasmine.

3.  Procédé pour la production de l'activateur de plasminogène I chaîne double selon la revendication 1, qui comprend l'incubation d'une fraction contenant l'activateur de plasminogène d'une liqueur mise en culture sur tissu de cellules dérivées de tissu humain normal à une température d'environ 10° C à environ 80° C dans son étape de séparation et de purification.

4.  Procédé selon la revendication 3, où la température est d'environ 20° C à environ 40° C.

5.  Procédé selon les revendications 3 ou 4, où l'étape d'incubation est effectuée pendant une période de temps d'environ 5 jours à environ 10 jours.

6.  Procédé selon l'une quelconque des revendications précédentes, où les cellules dérivées du tissu normal humain sont choisies dans le groupe consistant en cellules dérivées de reins intestins, poumons, coeur, urètre, peau ou prépuce d'embryon humain ou embryon humain entier ; cellules dérivées du placenta humain ; et cellules dérivées de rein, intestins, poumons, glande thyroïdienne, coeur, urètre ou peau d'être humain.

**Ansprüche**

1.  Verfahren zur Herstellung eines doppelkettigen Plasminogenaktivators I, das umfaßt, daß eine Fraktion, die den Plasminogenaktivator aus einer Gewebekulturflüssigkeit von Zellen menschlichen Normalgewebes enthält, beim Isolierungs- und Reinigungsschritt einer Enzymbehandlung unterworfen wird, oder daß ein gereinigter einkettiger Plasminogenaktivator II einer Enzymbehandlung unterworfen wird, wobei die Plasminogenaktivatoren I und II folgende Eigenschaften besitzen:

I

a) durch Gelfiltration bestimmte Molmasse:
63 000 ± 10 000;
b) isoelektrischer Punkt: 7,0 - 8,5;
c) Affinität zu Fibrin: vorhanden;
d) Affinität zu Concanavalin A: vorhanden;
e) optimaler pH: 7 - 9,5;
f) Verhalten bei Reduktion: Zersetzung;
g) hydrolytische Wirkung auf synthetische Substrate:

| Synthetisches Substrat | hydrolytische Wirkung (%)[1] |
|---|---|
| Pro-Phe-Arg-MCA | + |
| Z-Phe-Arg-MCA | - |
| Boc-Glu-Lys-Lys-MCA | - |
| Boc-Phe-Ser-Arg-MCA | ++++ |

| Synthetisches Substrat | hydrolytische Wirkung (%)[1] |
|---|---|
| Glt-Gly-Arg-MCA | ++ |
| Boc-Ileu-Glu-Gly-Arg-MCA | +++ |
| Boc-Val-Pro-Arg-MCA | + |
| Suc-Leu-Leu-Val-Thr-MCA | - |
| Boc-Leu-Ser-Thr-Arg-MCA | + |
| Boc-Leu-Thr-Arg-MCA | ++ |
| Suc-Ala-Ala-Pro-Phe-MCA | - |
| Boc-Val-Leu-Lys-MCA | + |
| Boc-Leu-Gly-Arg-MCA | ++++ |

[1] Es gilt folgende fünfstufige Einteilung, wobei die Berechnungen auf 1 $\mu$M Aminomethylcumarin als 100% bezogen sind:

- : <2%
+ : 2 - 30%
++ : 31 - 60%
+++ : 61 - 90%
++++ : >90%

II

a) durch Gelfiltration bestimmte Molmasse:
63 000 ± 10 000;
b) isoelektrischer Punkt: 7,0 - 8,5;

c) Affinität zu Fibrin: vorhanden;
d) Affinität zu Concanavalin A: vorhanden;
e) optimaler pH: 7 - 9,5;
f) Verhalten bei Reduktion: keine wesentliche Zersetzung;
g) hydrolytische Wirkung auf synthetische Substrate:

| Synthetisches Substrat | hydrolytische Wirkung (%)[1] |
|---|---|
| Pro-Phe-Arg-MCA | − |
| Z-Phe-Arg-MCA | − |

| Synthetisches Substrat | hydrolytische Wirkung (%)[1] |
|---|---|
| Boc-Glu-Lys-Lys-MCA | − |
| Boc-Phe-Ser-Arg-MCA | + |
| Glt-Gly-Arg-MCA | − |
| Boc-Ileu-Glu-Gly-Arg-MCA | + |
| Boc-Val-Pro-Arg-MCA | + |
| Suc-Leu-Leu-Val-Thr-MCA | − |
| Boc-Leu-Ser-Thr-Arg-MCA | − |
| Boc-Leu-Thr-Arg-MCA | + |
| Suc-Ala-Ala-Pro-Phe-MCA | − |
| Boc-Val-Leu-Lys-MCA | − |
| Boc-Leu-Gly-Arg-MCA | + |

[1] Es gilt folgende fünfstufige Einteilung, wobei die Berechnungen auf 1 $\mu$M Aminomethylcumarin als 100% bezogen sind:

− : <2%

+ : 2 - 30%

2. Verfahren nach Anspruch 1, wobei das Enzym Plasmin ist.

3. Verfahren zur Herstellung des doppelkettigen Plasminogenaktivators I nach Anspruch 1, das darin besteht, daß eine Fraktion, die den Plasminogenaktivator aus einer Gewebekulturflüssigkeit von Zellen menschlichen Normalgewebes enthält, beim Isolierungs- und Reinigungsschritt einer Inkubation bei einer Temperatur von etwa 10 °C bis etwa 80 °C unterworfen wird.

4. Verfahren nach Anspruch 3, wobei die Temperatur im Bereich von etwa 20 °C bis etwa 40 °C liegt.

5. Verfahren nach Anspruch 3 oder 4, wobei der Inkubationsschritt über einen Zeitraum von etwa 5 Tagen bis etwa 10 Tagen durchgeführt wird.

6. Verfahren nach irgendeinem der vorstehenden Ansprüche, wobei die Zellen des menschlichen Normalgewebes aus der Gruppe von Zellen ausgewählt werden, die gewonnen wurden aus humanembryonalen Nieren, Därmen, Lungen, Herzen, Harnleitern, Haut oder Vorhaut, oder aus einem ganzen Humanembryo, aus Humanplazenta-Zellen, sowie Zellen, die gewonnen wurden aus menschlichen Nieren, Därmen, Lungen, Schilddrüsen, Herzen, Harnleitern oder Haut.

# FIG. 1

# FIG. 2

Plasmin Treatment Time ( min. )